# EUROPEAN PATENT SPECIFICATION

(11) **EP 0 303 518 B1**
(45) Date of publication and mention of the grant of the patent: **17.02.1993**
(21) Application number: 88307546.7
(22) Date of filing: 15.08.1988
(51) Int. Cl.: C08F 220/06, A61L 15/00

(54) **Improved process for preparing water-absorbing resins**
Verfahren zur Herstellung von wasserabsorbierenden Harzen
Procédé de préparation de résines absorbant l'eau

(30) Priority: 14.08.1987 US 85974
(43) Date of publication of application: 15.02.1989
(73) Proprietor: AMERICAN COLLOID COMPANY, Arlington Heights, Illinois 60004 (US)
(72) Inventor: Alexander, William, Naperville Illinois 60540 (US); Anderson, Mark, Wheaton Illinois 60187 (US); Regan, Barbara R., Glenview Illinois 60025 (US)
(74) Representative: Wain, Christopher Paul

(56) References cited:
- EP-A- 0 207 714
- DE-A- 3 239 476

## Description

The present invention relates to a method and for manufacturing polyacrylate resins having improved water absorbing properties and more particularly to an improved process for preparing, either batch-wise, or continuously, cross-linked polymers of acrylic acid and a polyvinyl monomer having a new and unexpectedly low free acrylic monomer level. "Free monomer" or "free acrylic monomer", as used herein, includes any free acrylic acid in monomer form, as well as any acrylic monomer in the neutralized or salt form, which has not reacted to form a polymer.

Water absorbing resins have found wide use in sanitary goods, hygenic goods, water retaining agents, dehydrating agents, sludge coagulants, thickening agents, condensation preventing agents and release control agents for various chemicals. Water absorbing resins heretofore known include hydrolysis products of starch-acrylonitrile graft polymers, carboxymethyl-cellulose, cross-linked polyacrylate products and other resins such as polyvinyl alcohol, polyethylene oxide and polyacrylonitrile resins. Of these water absorbing resins, the hydrolysis products of starch and acrylonitrile graft polymers have comparatively high ability to absorb water but require a cumbersome process for production and have the drawbacks of low heat resistance and decaying or decomposing easily due to the presence of starch.

One of the processes for polymerizing acrylic acid and acrylates is aqueous solution polymerization. The polymer obtained by this process is soluble in water and, therefore, is cross-linked to modify the polymer into a useful water absorbing resin. However, even if the modification is effected by reacting a cross-linking agent concurrently with or after aqueous solution polymerization, the resulting reaction product is in the form of a highly viscous aqueous solution or a gel containing absorbed water which is difficult to handle. Thus, the aqueous solution or gel must be dehydrated (dried) to obtain a water absorbing resin in the desired solid or powder form. It is nevertheless difficult to dry the reaction product efficiently by the usual rotary drum roller method or spray drying method because care must be taken to avoid excessive cross-linking which results from overheating during drying, and insufficient drying results in reduced cross-linking density.

EP-A-207 714 discloses a process for preparing a solid water absorbing resin which comprises mixing a monomer solution of (A) acrylic acid, 70-100 mole percent neutralized; and (B) a water-miscible to water-soluble polyvinyl monomer; in a combined concentration of at least 30 wt. % with water to form a mixed monomer solution, and initiating polymerization of monomers (A) and (B) such that during polymerization, the exothermic heat of reaction is substantially the only heat energy used to accomplish polymerization, cross-linking and to drive off sufficient water to obtain a solid cross-linked resin having a water content of 15 percent by weight or less. As polymerization initiators redox initiators and thermal initiators are mentioned. All the examples use only one kind of initiator.

A problem associated with the polymerization of acrylic acid in the manufacture of water-absorbent resins as mentioned above is that the polymerization reaction does not proceed to substantial completion leaving a significant amount of free acrylic monomer in the polymer. Such unpolymerized monomer is undesirable in all circumstances and is wholly unacceptable for some uses of the water-absorbent resins, for example in disposable diapers.

We have found that by using a combination of two different kinds of polymerization initiator acrylic acid can be polymerized to form solid cross-linked resins having a very low content of free acrylic monomer, that is normally less than 1000 PPM and, in preferred embodiments of the method, less than 200 PPM of free acrylic monomer.

According to the present invention, there is provided a method of preparing a solid water absorbing resin which comprises mixing a monomer solution of (A) acrylic acid neutralized 70-100 mole percent; and (B) a water-miscible or water-soluble polyvinyl monomer in a combined concentration of from 30 to 80 wt. %, with water to form a mixed monomer solution, and adding a thermal initiator and a redox initiator to the mixed monomer solution to form an initiated mixed monomer solution, the mixed monomer solution being at a temperature which is below the decomposition temperature of the thermal initiator when the thermal initiator is added thereto, but which is high enough for the addition of the redox initiator to cause sufficient polymerization of the monomers to raise the temperature of the initiated mixed monomer solution to a level such that the thermal initiator decomposes sufficiently to provide, together with the redox initiator, substantially complete polymerization of the monomers.

Preferably during polymerisation the exothermic heat of reaction is substantially the only heat energy used to accomplish polymerization, cross linking and to drive off sufficient water to obtain a solid cross-linked resin having a water content of not more than 15 percent by weight.

This method can be carried out batchwise or continuously. In a preferred manner of operating the method continuously, the mixed monomer solution is combined with the thermal initiator and the redox initiator in a mixing chamber (48), the resulting mixture is supplied to a polymerization station (50) for polymerization, the polymerization station being simultaneously continuously supplied with additional mixed monomer solution and additional polymerization initiators, and solid polyacrylate resin is continuously removed from the polymerization station.

Preferably the mixed monomer solution and the polymerisation initiators are supplied to the mixing chamber (48) at rates such that the combined concentration of the initiators is continuously at least 0.5 wt. % with respect to the monomers (A) and (B).

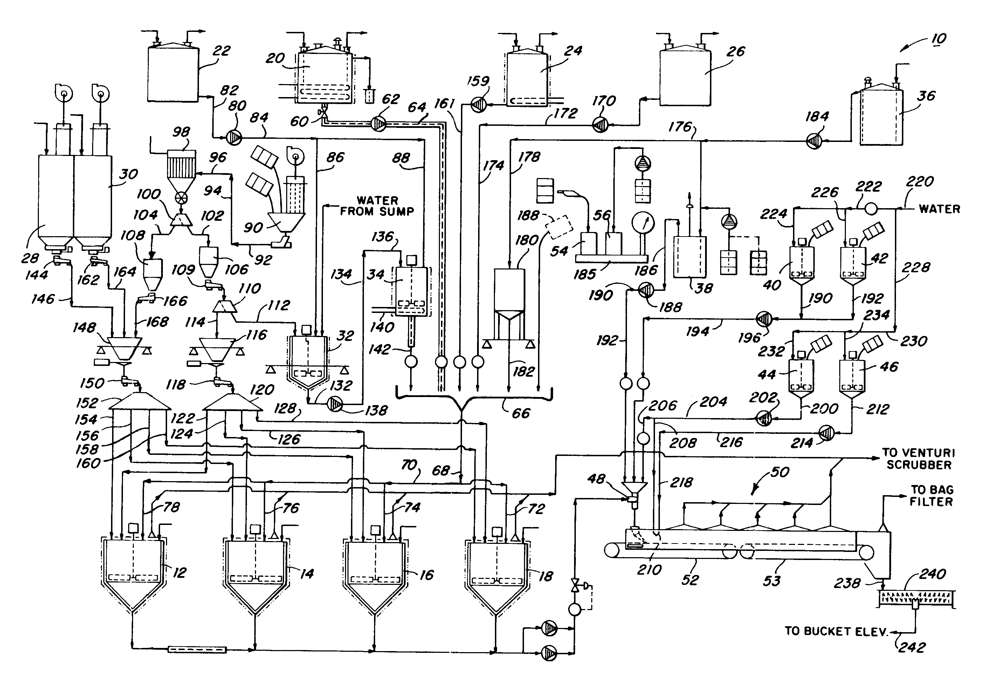

An installation suitable for carrying out a preferred form of the continuous method according to the invention is shown, by way of example, in the single Figure of the accompanying drawings which is a schematic representation of such installation.

As indicated it has been found that acrylic acid neutralized in the range of 70 to 100 mole percent will polymerize and cross-link rapidly with a polyvinyl monomer cross-linking agent to drive away excess water leaving a solid water absorbing resin having a desired degree of polymerization as well as new and unexpectedly low free monomer levels and water absorbing capacity. A combination of thermal and redox polymerization initiators is added to the aqueous monomer mixture to aid in polymerization and to reduce the free monomer content to unexpectedly low levels.

According to the present invention, an aqueous mixed monomer solution is prepared first comprising acrylic acid neutralized 70 to 100 mole percent, a water-miscible or water-soluble polyvinyl monomer, and water wherein the mixed monomer solution contains the acrylate monomer and the polyvinyl monomer in a combined concentration of 30 to 80 wt. %. The mixed monomer solution preferably contains 1 to 10 wt. % of an organic solvent based on the weight of monomers (A) and (B). The acrylic acid, acrylate and polyvinyl monomers are preferably present in the mixed monomer solution in a combined concentration of less than 70 wt. %, and more preferably less than 55 wt. %, of the monomer solution. The concentration of the monomers is deliberately determined considering the state of the solution (i.e. as to whether or not the monomers can be completely dissolved in water), ease of the reaction of the monomers, and escape of the monomers due to scattering during the reaction. The aqueous solution can be conveniently prepared by placing acrylic acid, a strong alkali such as potassium hydroxide and/or ammonium hydroxide or a basic amine for neutralizing the acid, and the polyvinyl monomer into water in such amounts that the resulting solution has the above-mentioned 30-80 wt. % monomer concentration. To dissolve the monomers thoroughly, the mixture can be heated to an elevated temperature. Any strongly basic alkali metal compound can be used for neutralization of the acrylic acid, such as potassium hydroxide, sodium hydroxide, lithium hydroxide, cesium hydroxide, potassium carbonate or sodium carbonate. Although it is desirable to use the neutralizing agent usually in an amount sufficient to neutralize acrylic acid 100 mole %, there is no particular need to neutralize the acid 100% insofar as the neutralizing agent, e.g., hydroxide, is used in such an amount as to achieve not less than about 70% neutralization. Accordingly, the aqueous solution may contain up to about 30% of free acrylic acid. However, a large quantity of free acrylic acid, if present in the aqueous solution, is likely to partly splash out of the system to result in a loss during the reaction, leading to a reduced degree of polymerization. Use of an excessive amount of the neutralizing agent will not raise any particular problem, but the excess does not participate in the polymerization reaction and is therefore useless.

It is preferred to use a combination of neutralizing agents, that is one which reacts endothermically with acrylic acid, e.g., a basic ammonium compound, such as ammonium carbonate and/or ammonium hydroxide, and one which reacts exothermically with acrylic acid, e.g., potassium hydroxide. This serves to maintain the monomer reactants in the mixed monomer solution at a proper temperature below the decomposition temperature of a thermal initiator without the necessity of cooling the reaction vessel.

The acrylic acid neutralized 70-100 mole percent is preferably mixed with the water-miscible or water-soluble polyvinyl monomer in an aqueous solution at a temperature of from 30° to 85°C, more preferably 45° to 55°C, prior to the addition of the initiators.

The temperature of the mixed monomer solution can vary considerably in accordance with the process of the present invention prior to the addition of the thermal and redox initiators, depending upon the particular thermal initiator added. In any event, the initial temperature of the mixed monomer solution should be below the temperature at which the thermal initiator decomposes or otherwise causes substantial polymerization initiation and the temperature of the mixed monomer solution should be high enough that the redox initiator causes sufficient polymerization at the initial temperature of the mixed monomer solution to raise the temperature of the mixed monomer solution to a level sufficient that the thermal initiator, together with the redox initiator, causes substantially complete polymerization, that is leaving less than 1000 PPM free monomer. Free monomer levels less than 200 PPM and even less than 100 PPM have been achieved in accordance with the process of the present invention. The combination of initiators can be added to the mixed monomer solution in a batch process, or continuously as shown in the drawing.

In accordance with the continuous process shown in the drawing, the mixed monomer solution is subjected to a polymerization reaction and a cross-linking reaction by the continuous addition of a combination of polymerization initiators in an in-line mixing device just prior to depositing the initiated mixed monomer solution onto an endless moving conveyor belt. The polymerization reaction begins within a very short period of time, e.g., about 30 seconds, as a result of the redox initiator. Once the polymerization reaction begins, the exothermic heat of reaction raises the temperature of the mixed monomer solution to a degree that the thermal initiator also initiates further polymerization to substantial completion. If the monomer concentration is at least 30 wt. % of the aqueous monomer mixture, the heat of the polymerization and cross-linking reactions will evaporate water rapidly from the reaction product as it travels down the endless belt to form a dry solid (less than 15 percent by weight water) water absorbing resin without the need for any subsequent drying step. The solid can be easily pulverized into a powder suitable for any desired use.

In operating the process continuously, a hot, i.e. at least 85° F (30°C), aqueous solution is prepared first including acrylic acid neutralized 70 to 100 mole percent, optionally acrylamide, a water-miscible or water-soluble polyvinyl monomer, and water in one or more mixed monomer solution storage vessels. A thermal initiator and a redox initiator are stored in separate initiator storage vessels so that the reactants from the mixed monomer vessel and each of two initiator vessels are fed simultaneously into an in-line mixing device disposed in close proximity to a traveling endless conveyor belt. The aqueous solution can be prepared easily by placing (i) acrylic acid, and an amine, and/or a caustic alkali and/or ammonia for neutralizing the acid; (ii) acrylamide (0-30 mole percent); and (iii) a polyvinyl monomer into water to form a mixed monomer solution. To dissolve the monomers thoroughly, the mixture can be heated to an elevated temperature up to 85°C, and is more preferably at a temperature of from 45 to 55°C.

The polyvinyl monomer to be used in the invention should be miscible with or soluble in water so that the monomer will be uniformly dissolved or dispersed in the aqueous solution of the monomer mixture. Examples of such polyvinyl monomers include bisacrylamides, such as N,N'-methylenebisacrylamide and N,N'-methylenebismethacrylamide; polyacrylic (or polymethacrylic) acid esters represented by the following formula (I); and diacrylamides represented by the following formula (II). Among these, especially preferably are N,N'-methylenebisacrylamide, and N,N'-methylenebismethacrylamide.

### Formula (I)

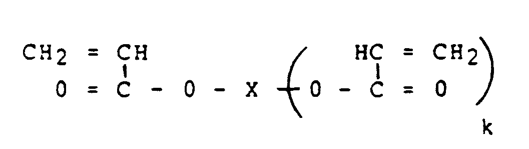

wherein X is ethylene, propylene, trimethylene, hexamethylene, 2-hydroxypropylene, (CH₂CH₂O)ₙCH₂CH₂ - or 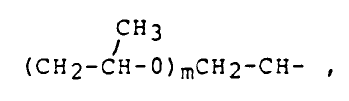
n and m are each an integer of from 5 to 40, and k is 1 or 2.

The compounds of formula (I) are prepared by reacting polyols, such as ethylene glycol, propylene glycol, trimethylolpropane, 1,6-hexanediol, glycerin, pentaerythritol, polyethylene glycol and polypropylene glycol, with acrylic acid or methacrylic acid.

### Formula (II):

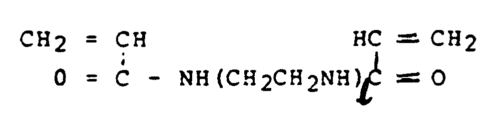

wherein ℓ is 2 or 3.

The compounds of the formula (II) are obtained by reacting polyalkylenepolyamines, such as diethylenetriamine and triethylenetetramine, with acrylic acid.

The polyvinyl monomer is preferably used in an amount of from 0.001 to 0.6 wt. % of the amount of acrylic monomers in the aqueous monomer mixture. More preferably, however, the polyvinyl monomer is present in the aqueous solution in an amount of at least 0.2 wt. % based on the total weight of monomers to provide a resin sufficiently cross-linked to have a "dry feel" after significant water absorption. If the polyvinyl monomer is included in the aqueous solution in an amount of 0.2 to 0.6 weight percent based on the weight of neutralized acrylic acid and polyvinyl monomers, the resulting polymer will have an exceedingly "dry feel" after significant absorption of water.

The aqueous mixed monomer solution is maintained at a desired temperature, depending upon the temperature at which the thermal initiator decomposes or otherwise becomes effective, and the degree to which the exothermic heat of reaction causes the temperature of the mixed monomer solution to rise as a result of the initiation by the redox initiator. The temperature of the mixed monomer solution should be high enough that the redox initiator causes the mixed monomer solution to rise in temperature to a degree that the thermal initiator decomposes or otherwise effectively continues the polymerization reaction. The temperature of the mixed monomer solution is preferably maintained at from 100° F. to 145° F. (37.8-62.8°C), and in accordance with the preferred thermal initiators disclosed hereinafter, more preferably at from 45° to 55°C.

Suitable redox initiators for use in the method of the invention include, for example, a reducing agent, such as a sulfite or bisulfite of an alkali metal, such as ammonium sulfite, ammonium bisulfite, or ammonium metabisulfite; a persulfate of an alkali metal or ammonium persulfate; t-butyl hydroperoxide; di-t-butyl hydroperoxide; t-butyl perbenzoate; t-butyl peroxy isopropyl carbonate; and peroxy-3,3,5 trimethylcyclohexane. These redox initiators can be used singly or in a suitable combination. Of these, a redox initiator comprising a combination of ammonium persulfate and sodium hydrogensulfite is particularly preferred. These initiators are advantageously used in the form of an aqueous solution but can be dissolved in a suitable solvent, e.g., alcohol, if insoluble in water. The redox initiator is used in an amount, calculated as solids, of from 0.1 to 10%, preferably 0.5 to 5%, of the combined weight of the monomers, namely acrylate (and free acrylic acid), acrylamide, and polyvinyl monomer. Depending on the amount and kind of the initiator, the initiator is usable together with isopropyl alcohol, alkylmercaptan or other chain transfer agents to control the molecular weight of the polyacrylate to be obtained.

Examples of suitable thermal initiators include azo initiators, such as azobisisobutyronitrile; 4-t-butylazo-4'-cyanovaleric acid; 4,4'-azobis(4-cyanovaleric acid); 2,2'-azobis(2-amidinopropane)dihydrochloride; 2,2'-azobis(2,4-dimethylvaleronitrile); dimethyl 2,2'-azobisisobutyrate; 2,2'-azobis(2,4-dimethylvaleronitrile); (1-phenylethyl)azodiphenylmethane; 2,2'-azobis(2-methylbutyronitrile); 1,1'-azobis(1-cyclohexanecarbonitrile); 2-(carbamoylazo)isobutyronitrile; 2,2'-azobis(2,4,4-trimethylpentane); 2-phenylazo-2,4-dimethyl-4-methoxyvaleronitrile; 2,2'-azobis(2-methylpropane); 2,2'-azobis(N,N'-dimethyleneisobutyramidine)dihydrochloride; 2,2'-azobis(N,N'-dimethyleneisobutyramidine; 4,4'-azobis(4-cyanopentanoic acid); 2,2'-azobis(2-methyl-N-[1,1-bis(hydroxymethyl)-2-hydroxy-ethyl]propionamide); 2,2'-azobis(2-methyl-N-[1,1-bis(hydroxymethyl)ethyl]propionamide); 2,2'-azobis[2-methyl-N-(2-hydroxyethyl)propionamide]; 2,2'-azobis(isobutyramide)dihydrate.

By the continuous addition of the combination of polymerization initiators, the mixed monomer solution is subjected to continuous polymerization at a polymerization station, such as on a surface of a liquid-impermeable conveyor belt or in a suitable reaction vessel, with evaporation of water without heating the system from outside. To achieve the full advantage of the present invention, the reaction is carried out by adding the combination of initiators, from separate initiator storage vessels, into the mixed monomer solution in a suitable in-line mixing device and causing the resulting initiated mixture to flow down onto and spread over a traveling conveyor belt. Alternatively, the initiators can be added to the mixed monomer solution as it is poured onto the conveyor belt.

The polymerization proceeds rapidly after admixing the initiators with the mixed monomer solution and is completed within a short period of time, usually in about 30 seconds to about 10 minutes. The reaction is exothermic, so that the reactants are rapidly heated from a reaction temperature of at least 30°C to about 130°C (265° F) by the heat of polymerization. Consequently, particularly where the monomer concentration in the mixed solution is at least 50 wt. %, the water evaporates from the system rapidly to give a relatively dry, solid polymer of low water content without resorting to any external heating or other further treatment. The water content of the polymer is usually not more than 15%, and generally from 8 to 12% by weight, as recovered. Subsequently, the dry solid polymer can readily be reduced to a powder, for example by pulverization, without a drying step.

Polystyrene and/or methylcellulose can be added to the mixed monomer solution in an amount of 0.5 to 10 % based on the total weight of monomers in the mixed monomer solution to increase the porosity and water absorbing capacity of the polymers. It has been found, quite surprisingly, that polystyrene and methylcellulose will substantially increase the water absorbing capacity of the resin described herein. To achieve the full advantage of the present invention, the polystyrene and methylcellulose should be added in an average grain size of not more than 5 micrometers.

The water-absorbing polymer powder thus obtained has outstanding water absorbing ability, an unexpectedly low free monomer level, is useful for sanitary goods, paper diapers, disposable diapers and like hygenic goods, agricultural or horticultural water retaining agents, industrial dehydrating agents, sludge coagulants, thickening agents, condensation preventing agents for building materials, release control agents for chemicals and various other applications.

The present invention will be described in greater detail with reference to the drawing and the following examples.

Referring now to the drawing, there is illustrated a method and apparatus for the continuous manufacture of water absorbing resin, generally designated by reference numeral 10.

Generally, the apparatus 10 includes a plurality of monomer solution mixing vessels 12, 14, 16 and 18; an acrylic acid storage vessel 20, one or more neutralizing agent storage vessels such as potassium hydroxide storage vessel 22, sodium hydroxide storage vessel 24, ammonium hydroxide storage vessel 26, sodium carbonate storage vessel 28, potassium carbonate storage vessel 30 and neutralizing agent mixing vessels 32 and 34; one or more second monomer storage vessels, such as for acrylamide and styrene, 36 and 38; a plurality of thermal initiator storage vessels 40 and 42; a plurality of redox initiator storage vessels 44 and 46; an in-line mixer or blender 48 for blending the initiators with the mixed monomer solution; and a polymerization station, generally designated 50, including a pair of endless traveling conveyor belts 52 and 53, and a plurality of cross-linking agent storage vessels 54 and 56.

Acrylic acid solution from acrylic acid storage vessel 20, maintained at about 55-75° F. (13°-24°C) flows through valve 60 and pump 62 through insulated supply conduit 64 to a combining station 66 where the acrylic acid is combined with one or more neutralizing agents, such as potassium hydroxide from storage vessel 22, sodium hydroxide from storage vessel 24, and/or ammonium hydroxide from storage vessel 26 and may be combined with one or more other monomers such as acrylamide and/or styrene from second monomer storage vessels 36 and/or 38. The acrylic acid solution together with the aqueous solutions of one or more neutralizing agents and, optionally, one or more other monomers for co-polymerization, are combined and fed through monomer solution feed conduits 68, 70, 72, 74, 76, and 78 to the four monomer solution mixing vessels 12, 14, 16 and 18. The monomer mixing vessels 12, 14, 16 and 18 are filled in a staggered time sequence such that one monomer mixing vessel, e.g., 12, is completely mixed and ready for transport to the in-line blender 48 while the other monomer solution mixing vessels, e.g., 14, 16 and 18, are being filled such that after monomer mixing vessel 12 is depleted, one of the other monomer mixing vessels 14, 16 or 18 is sufficiently mixed and homogenized such that it is ready for transport of the mixed monomer solution to the blender 48 to maintain a continuous flow of mixed monomer solution to the blender 48 for continuous operation. In this manner, a continuous supply of mixed monomer solution is always ready for transport to the blender 48 to maintain the process continuously.

The potassium hydroxide solution stored in neutralizing agent storage vessel 22 is received, as supplied, at about a 45% by weight concentration as an aqueous solution and is pumped through pump 80 and conduits 82, 84, 86 and 88 to one or more potassium hydroxide storage vessels 32 and/or 34 where the potassium hydroxide solution can be further concentrated, for example to a preferred level of about 56% potassium hydroxide by weight, by the addition of a more concentrated form of potassium hydroxide, such as 100% solid potassium hydroxide, through hopper 90.

In accordance with the preferred apparatus, as shown in the drawing, either potassium hydroxide or ammonium carbonate can be added to the hopper 90 and the neutralizing agents conveyed through conduits 92, 94 and 96 to a vacuum separator 98 where the neutralizing agents are conveyed by diverter 100 to either a concentrated potassium hydroxide supply line 102 and concentrated potassium hydroxide storage vessel 106 or to a concentrated ammonium carbonate supply line 104 and concentrated ammonium carbonate storage vessel 108, respectively. The concentrated potassium hydroxide from vessel 106 is conveyed by a vibratory feeder 109 to diverter 110 where some of the concentrated potassium hydroxide, e.g., in solid form, is transported through conduit 112 to the potassium hydroxide tank 32 to increase the potassium hydroxide concentration to a desired level, e.g., 56% by weight potassium hydroxide. The diverter 110 conveys some of the potassium hydroxide through conduit 114 to a weigh hopper 116 such that some of the concentrated potassium hydroxide can be conveyed by vibratory feeder 118 to a turn head 120 for further, straight addition of potassium hydroxide to the monomer mixing vessels 12, 14, 16 and 18 through conduits 122, 124, 126 and 128.

The more concentrated aqueous solution of potassium hydroxide in vessel 32 is conveyed through conduits 132, 134 and 136 and pump 138 to the potassium hydroxide storage vessel 34 where the potassium hydroxide is stored at a suitable temperature, such as 65° F. (18.3°C), through an internal heat transfer water supply line 140. The more concentrated potassium hydroxide from vessel 34 is fed to the mixing station 66 through an insulated supply conduit 142. Sodium carbonate from neutralizing agent supply vessel 28 can be supplied to the monomer solution mixing vessels 12, 14, 16 and/or 18 by vibratory feeder 144, conduit 146, weigh hopper 148, vibratory feeder 150 and turn head 152 through conduits 154, 156, 158 and 160. Sodium hydroxide can be conveyed directly from the sodium hydroxide storage vessel 24 through pump 159 and conduit 161 to the combining station 66. Optionally, the sodium carbonate can be substituted with or combined with potassium carbonate from neutralizing agent storage vessel 30, vibratory feeder 162 and conduit 164 leading to weigh hopper 148; or the sodium carbonate can be substituted for and/or combined with ammonium carbonate from ammonium carbonate vessel 108, vibratory feeder 166 and conduit 168 leading to weigh hopper 148, such that any desired neutralizing agent in any desired concentration can be fed directly to the monomer solution mixing vessel 12, 14, 16 and/or 18 through the conduits 154, 156, 158 and 160. Ammonium hydroxide from ammonium hydroxide storage vessel 26 optionally can be fed to the mixing station 66 and then to the monomer solution mixing vessels 12, 14, 16 and 18, as described, by feeding the ammonium hydroxide solution through pump 170 and conduits 172 and 174 to the mixing station 66, where the neutralizing agents are combined with the acrylic acid solution, and optionally, one or more other monomers from monomer storage vessel 36 or 38, monomer supply conduits 176 and 178, monomer weigh tank 180 and conduit 182 by pump 184. Optionally, additional monomer for co-polymerization with acrylic acid can be supplied directly to the blender 48 for monomer storage vessel 38 through conduit 186, pump 188 and conduits 190 and 192. In this manner, any desired level of and combination of neutralizing agents and monomers can be supplied to the blender 48 for initiation and polymerization.

One or more cross-linking agents are stored in cross-linking agent storage vessels 54 and 56 where the cross-linking agent is weighed on scale 186 and fed manually, as indicated diagrammatically at 188, to the mixing station 66 for supply to the monomer solution mixing vessels 12, 14, 16 and 18.

In accordance with an important feature of the present invention, one or more thermal initiators are stored in the thermal initiator storage vessels 40 and 42 are are delivered directly to the in-line blender 48 through conduits 190, 192 and 194 and pump 196 and, simultaneously, one or more redox initiators are supplied to the blender 48 through redox initiator storage vessel 44, conduit 200, pump 202 and conduits 204 and 206 for initiation of the mixed monomer solution. Optionally some of the redox initiator from redox initiator storage tank 44 can be fed onto the surface of the mixed monomer solution as it travels on the liquid-impermeable endless conveyor belt 52 directing some of the initiator through conduit 208 directly onto the surface of the polymerizing mixed monomer solution, such as at spray head 210. Conveyor belt 53 is air-permeable to allow air to circulate over and through the polymerized and polymerizing acrylic acid and neutralized acrylic acid, as well as any other polymerized and polymerizing monomer delivered to the impermeable, first belt 52. The same or different redox initiator can be fed directly onto the surface of the polymerizing mixed monomer solution from initiator storage vessel 46 through conduit 212, pump 214 and conduits 216 and 218 through the spray head 210.

In accordance with another important feature of the present invention, a different, polymerization starting redox initiator is held in initiator storage vessel 46 which will initiate the mixed monomer solution at the temperature that the monomer solution has attained when it exits the in-line mixer 48, generally at the storage temperature of the mixed monomer solution in the monomer solution mixing vessels 12, 14, 16 and 18. Once the mixed monomer solution begins to polymerize as a result of the redox initiator from storage vessel 46, sufficient exothermic heat of reaction will maintain the polymerization reaction without further addition of the redox initiator from the initiator storage vessel 46. It has been found that sodium thiosulfate stored in redox initiator storage vessel 46 initiates the acrylic acid polymerization at a sufficiently low temperature that it can be used to start the polymerization reaction and, thereafter, is no longer needed once the polymerization has begun. Optionally, water can be supplied to the thermal initiator storage vessels 40 and 42 and to the redox initiator storage vessels 44 and 46 for diluting the initiator concentration from water supply line 220 and conduits 222, 224, 226, 228, 230, 232 and 234.

The polymerized dry (less than about 15% by weight water) polymer exits conveyor belts 53 through conduit 238 into a screw or auger discharge screw device 240 and is directed out of the discharge screw 240 through conduit 242 to a bucket elevator. The polymer then can be ground to any appropriate particle size distribution.

### EXAMPLE 1

2,530 pounds (1147.6 kgs) of acrylic acid solution from acrylic acid storage vessel 20 at 110° F. (43.3°C) and a concentration of 99% by weight flows through valve 60 and pump 62 through conduit 64 to a combining station 66 to vessel 12, 14, 16 or 18. Then 410 (186 kg) pounds of styrene from second monomer storage vessel 36 flows through pump 184 through conduits 176 and 178 to monomer weigh tank 180 and is charged to combining station 66 and then to vessel 12, 14 16 or 18, together with the acrylic acid.

1,700 pounds (771.1 kg) of a 45% by wt. potassium hydroxide is pumped from storage vessel 22, through pump 80, conduits 82, 84 86 and 88 to one or both potassium hydroxide storage solution is further concentrathe potassium hydroxide solution is further concentrated to a concentration of 53% by wt. and is pumped from vessel 32 through conduits 132, 134 and 136 and pump 138 to potassium hydroxide storage vessel 34 where the potassium hydroxide can be stored before transfer to mixing station 66 through the insulated supply conduit 142 and finally to vessel 12, 14, 16 or 18.

650 pounds (294.8 kg) of potassium carbonate is supplied from vessel 28 to monomer solution mixing vessels 12, 14, 16 and/or 18 by vibratory feeder 144, through conduit 146, weigh hopper 148, vibratory feeder 150 and turn hed 152 through conduit 154, 156, 158 or 160.

2.9 pounds (1.32 kg) of N, N-methylenebisacrylamide cross-linking agent is weighed on scale 186 and fed manually as indicated diagrammatically at 188 to mixing station 66 for supply to the monomer solution mixing vessel 12, 14, 16 or 18, where the mixed monomer solution is agitated at 110° F. (43.3 C)

The neutralized monomer from the monomer solution mixing vessel 12, 14, 16 or 18 is delivered to the impermeable first belt 52 through in-line mixer 48 where it is combined with a redox initiator and, a thermal initiator raising the temperature of the mixed monomer solution to 108° F (42.5°C), which is above the decomposition temperature of the thermal initiator. The thermal initiator is stored in thermal initiator storage vessels 40 and 42 consisting of a 12% solution of 2,2′-azobis-(2-amidinopropane) hydrochloric acid. 72.2 pounds (32.75 kg) of thermal initiator would be used for the above batch. the initiator is delivered to the in-line blender 48 through conduit 190, 192 and 194 and pump 199 and, simultaneously 24.1 pounds (10.93 kg) of 33% ammonium persulfate redox initiator is supplied to the blender 48 through redox initiator storage vessel 44, conduit 200, pump 202 and conduits 204 and 206 for the initiation of the mixed monomer solution.

3.0 pounds (1.36 kg) of a 33% by wt. solution of a 50/50 mixture of sodium thio sulfate and ammonia persulfate redox initiators stored in vessel 44 is fed onto the surface of the polymerizing mixed monomer solution, such as a spray head 210.

### EXAMPLE 2

2,940 pounds (132.56 kg) of acrylic acid solution from acrylic acid storage vessel 20 at 85° F (29.4°C) and a concentration of 99% by weight flows through valve 60 and pump 62 through conduit 64 to a combining station 66 to vessel 12, 14, 16 or 18.

1,700 pounds (771.1 kg) of a 45% by wt. potassium hydroxide solution is pumped from storage vessel 22, through pump 80, conduits 82, 84 and 88 to potassium hydroxide storage vessel 34 where the potassium hydroxide solution is stored. The potassium hydroxide solution is supplied from vessel 34 through conduit 142 and finally to vessel 12, 14, 16 or 18.

650 pounds (294.84 kg) of ammonium hydroxide is supplied from vessel 26 to monomer solution mixing vessels 12, 14, 16 and/or 18 by pump 170, through conduits 172 and 174.

2.9 pounds (1.32 kg) of N, N-methylenebisacrylamide cross-linking agent is weighed on scale 186 and fed manually as indicated diagrammatically at 188 to mixing station 66 for supply to the monomer solution mixing vessel 12, 14, 16 or 18, where the mixed monomer solution is mixed at 110° F. (43.3°C)

The neutralized monomer from the monomer solution mixing vessel 12, 14, 16 or 18 is delivered to the impermeable first belt 52 through in-line mixer 48 where it is combined with a thermal initiator and a redox initiator. The redox initiator raises the temperature of the mixed monomer solution to 150° F. (65.5°C) The thermal initiator is stored in thermal initiator storage vessels 40 and 42 consisting of a 12% solution of 2,2′-azobis-(2-amidinopropane) hydrochloride. 72.2 pounds (32.75 kg) of thermal initiator would be used for the above batch. The initiator is delivered to the in-line blender 48 through conduit 190, 192 and 194 and pump 199 and, simultaneously 24.1 pounds (10.93 kg) of 33% ammonium sulfite redox initiator is supplied to the blender 48 through redox initiator storage vessel 44, conduit 200, pump 202 and conduits 204 and 206 for the initiation of the mixed monomer solution.

3.0 pounds (1.36 kg) of a 33% by wt. solution of a 50/50 mixture of sodium thio sulfate and ammonia persulfate redox initiators stored in vessel 44 is fed onto the surface of the polymerizing mixed monomer solution, such as a spray head 210. After the monomer begins polymerization, the redox initiator is shut off at spray head 210.

### EXAMPLE 3

2,600 pounds (1179.3 kg) of acrylic acid solution from acrylic acid storage vessel 20 at 100°F and a concentration of 99% by weight flows through valve 60 and pump 62 through conduit 64 to a combining station 66 to vessel 12, 14, 16 or 18.

1,600 pounds (725.75 kg) of a 45% by wt. potassium hydroxide solution is pumped from storage vessel 22, through pump 80, conduits 82, 84 and 88 to potassium hydroxide storage vessel 32 where the potassium hydroxide solution is further concentrated to a concentration of 53% by wt. and is pumped from vessel 32 through conduits 132, 134 and 136 and pump 138 to potassium hydroxide storage vessel 34 where the potassium hydroxide can be stored before transfer to mixing station 66 through the insulated supply conduit 142 and finally to vessel 12, 14, 16 or 18.

750 pounds (340.19 kg) of sodium hydroxide is supplied from vessel 24 to monomer solution mixing vessels 12, 14, 16 and/or 18 by pump 159, through conduit 161.

2.9 pounds (132 kg) of diethylenetriamine-diacrylamide cross-linking agent is weighed on scale 186 and fed manually as indicated diagrammatically at 188 to mixing station 66 for supply to the monomer solution mixing vessel 12, 14, 16 or 18, where the mixed monomer solution is mixed at 110° F.

The neutralized monomer from the monomer solution mixing vessel 12, 14, 16 or 18 is delivered to the impermeable first belt 52 through in-line mixer 48 where it is combined with a thermal initiator and a redox initiator. The redox initiator raises the temperature of the mixed monomer solution to 150° F. (655°C) The thermal initiator is stored in thermal initiator storage vessels 40 and 42 consisting of a 12% solution of aziobisisobutyronitrile. 72.2 pounds (32.75 kg) of thermal initiator would be used for the above batch. The initiator is delivered to the in-line blender 48 through conduit 190, 192 and 194 and pump 199 and, simultaneously 24.1 pounds of 33% ammonium bisulfite persulfate redox initiator is supplied to the blender 48 through redox initiator storage vessel 44, conduit 200, pump 202 and conduits 204 and 206 for the initiation of the mixed monomer solution.

3.0 pounds (136 kg) of 33% by wt. solution of a 50/50 mixture of sodium thio sulfate and ammonia persulfate redox initiators stored in vessel 44 is fed onto the surface of the polymerizing mixed monomer solution, such as a spray head 210.

### EXAMPLE 4

3,000 pounds (1360.77 kg) of acrylic acid solution from acrylic acid storage vessel 20 at 110° F (43.3°C) and a concentration of 99% by weight flows through value 60 and pump 62 through conduit 64 to a combining station 66 to vessel 12, 14, 16 or 18, where the mixed monomer solution is mixed at 110° F. (43.3 C)

1,750 pounds (793.79 kg) of a 45% by wt. potassium hydroxide solution is pumped from storage vessel 22, through pump 80, conduits 82, 84 86 and 88 to one or both potassium hydroxide storage vessels 32 and 34 where the potassium hydroxide solution is further concentrated to a concentration of 53% by wt. and is pumped from vessel 32 through conduits 132, 134 and 136 and pump 138 to potassium hydroxide storage vessel 34 where the potassium hydroxide can be stored before transfer to mixing station 66 through the insulated supply conduit 142 and finally to vessel 12, 14, 16 or 18.

650 pounds (294.84 kg) of potassium carbonate is supplied from vessel 28 to monomer solution mixing vessels 12, 14, 16 and/or 18 by vibratory feeder 144, through conduit 146, weigh hopper 148, vibratory feeder 150 and turn hed 152 through conduit 154, 156, 158 or 160.

2.9 pounds (1.32 kg) or an ethylene glycol diallylester cross-linking agent is weighed on scale 186 and fed manually as indicated diagrammatically at 188 to mixing station 66 for supply to the monomer solution mixing vessel 12, 14, 16 or 18, where the mixed monomer solution is mixed at 110° F. (43.3°C)

The neutralized monomer from the monomer solution mixing vessel 12, 14, 16 or 18 is delivered to the impermeable first belt 52 through in-line mixer 48 where it is combined with a thermal initiator and a redox initiator. The redox initiator raises the temperature of the mixed monomer solution to 150° F. (65.5°C) The thermal initiator is stored in thermal initiator storage vessels 40 and 42 consisting of a 10% solution of 4-t-butylazo-4′-cyanovaleric acid. 72.2 pounds (32.75 kg) of thermal initiator would be used for the used batch. The initiator is delivered to the in-line blender 48 through conduit 190, 192 and 194 and pump 199 and, simultaneously 24.1 pounds of 33% t-butylhydroproxide redox initiator is supplied to the blender 48 through redox initiator storage vessel 44, conduit 200, pump 202 and conduits 204 and 206 for the initiation of the mixed monomer solution.

3.0 pounds (1.36 kg) of a 33% by wt. solution of a 50/50 mixture of sodium thio sulfate and ammonia persulfate redox initiators stored in vessel 44 is fed onto the surface of the polymerizing mixed monomer solution, such as a spray head 210.

### EXAMPLE 5

2,500 pounds (1133.98 kg) of acrylic acid solution from acrylic acid storage vessel 20 at 115° F (46.1°C) and a concentration of 99% by weight flows through valve 60 and pump 62 through conduit 64 to a combining station 66 to vessel 12, 14, 16 or 18. Then 400 pounds (181.44 kg) of styrene from second monomer storage vessel 36 flows through pump 184 through conduits 176 and 178 to monomer weigh tank 180 and is charged to combining station 66 and then to vessel 12, 14 16 or 18, together with the acrylic acid.

1,600 pounds (725.75 kg) of a 45% by wt. potassium hydroxide solution is pumped from storage vessel 22, through pump 80, conduits 82, 84 86 and 88 to one or both potassium hydroxide storage vessels 32 and 34 where the potassium hydroxide solution is further concentrated to a concentration of 53% by wt. and is pumped from vessel 32 through conduits 132, 134 and 136 and pump 138 to potassium hydroxide storage vessel 34 where the potassium hydroxide can be stored before transfer to mixing station 66 through the insulated supply conduit 142 and finally to vessel 12, 14, 16 or 18.

650 pounds (294.84 kg) of potassium carbonate is supplied from vessel 28 to monomer solution mixing vessels 12, 14, 16 and/or 18 by vibratory feeder 144, through conduit 146, weigh hopper 148, vibratory feeder 150 and turn hed 152 through conduit 154, 156, 158 or 160.

2.9 pounds (1.32 kg) of polyethylene glycol diacrylate cross-linking agent is weighed on scale 186 and fed manually as indicated diagrammatically at 188 to mixing station 66 for supply to the monomer solution mixing vessel 12, 14, 16 or 18, where the mixed monomer solution is mixed at 110° F. (43.3°C)

The neutralized monomer from the monomer solution mixing vessel 12, 14, 16 or 18 is delivered to the impermeable first belt 52 through in-line mixer 48 where it is combined with a thermal initiator and a redox initiator. The redox initiator raises the temperature of the mixed monomer solution to 150° F. (65.5°C) The thermal initiator is stored in thermal initiator storage vessels 40 and 42 consisting of a 12% solution of 2,2′-azobis(2,4-dimethylvaleronitrile. 72.2 pounds (32.75 kg) of thermal initiator would be used for the above batch. The initiator is delivered to the in-line blender 48 through conduit 190, 192 and 194 and pump 199 and, simultaneously 24.1 pounds (1093 kg) of 33% di-t-butyl hydroperoxide redox initiator is supplied to the blender 48 through redox initiator storage vessel 44, conduit 200, pump 202 and conduits 204 and 206 for the initiation of the mixed monomer solution.

3.0 pounds (1.36 kg) of a 33% by wt. solution of a 50/50 mixture of sodium thio sulfate and ammonia persulfate redox initiators stored in vessel 44 is fed onto the surface of the polymerizing mixed monomer solution, such as a spray head 210.

### EXAMPLE 6

2,530 pounds (1147.59 kg) of acrylic acid solution from acrylic acid storage vessel 20 at 100° F (37.8°C) and a concentration of 99% by weight flows through valve 60 and pump 62 through conduit 64 to a combining station 66 to vessel 12, 14, 16 or 18. Then 410 pounds (185.97 kg) of styrene from second monomer storage vessel 36 flows through pump 184 through conduits 176 and 178 to monomer weigh tank 180 and is charged to combining station 66 and then to vessel 12, 14 16 or 18, together with the acrylic acid.

1,700 pounds (771.11 kg) of a 45% by wt. potassium hydroxide solution is pumped from storage vessel 22, through pump 80, conduits 82, 84 86 and 88 to one or both potassium hydroxide storage vessels 32 and 34 where the potassium hydroxide solution is further concentrated to a concentration of 53% by wt. and is pumped from vessel 32 through conduits 132, 134 and 136 and pump 138 to potassium hydroxide storage vessel 34 where the potassium hydroxide can be stored before transfer to mixing station 66 through the insulated supply conduit 142 and finally to vessel 12, 14, 16 or 18.

650 pounds (294.84 kg) of potassium carbonate is supplied from vessel 28 to monomer solution mixing vessels 12, 14, 16 and/or 18 by vibratory feeder 144, through conduit 146, weight hopper 148, vibratory feeder 150 and turn hed 152 through conduit 154, 156, 158 or 160.

2.9 pounds (1.32 kg) of N, N-methylenebisacrylamide cross-linking agent is weighed on scale 186 and fed manually as indicated diagrammatically at 188 to mixing station 66 for supply to the monomer solution mixing vessel 12, 14, 16 or 18, where the mixed monomer solution is mixed at 110° F. (43.3°C)

The neutralized monomer from the monomer solution mixing vessel 12, 14, 16 or 18 is delivered to the impermeable first belt 52 through in-line mixer 48 where it is combined with a thermal initiator and a redox initiator. The redox initiator raises the temperature of the mixed monomer solution to 150° F. (65.5°C) The thermal initiator is stored in thermal initiator storage vessels 40 and 42 consisting of a 12% solution of dimethyl 2,2′-azobisisobutyrate. 72.2 pounds of thermal initiator would be used for the above batch. The initiator is delivered to the in-line blender 48 through conduit 190, 192 and 194 and pump 199 and, simultaneously 24.1 pounds of 33% t-butylperbenzoate redox initiator is supplied to the blender 48 through redox initiator storage vessel 44, conduit 200, pump 202 and conduits 204 and 206 for the initiation of the mixed monomer solution.

### EXAMPLE 7

2,530 pounds (1147.59 kg) of acrylic acid solution from acrylic acid storage vessel 20 at 115°F (46.1°C) and a concentration of 99% by weight flows through valve 60 and pump 62 through conduit 64 to a combining station 66 to vessel 12, 14, 16 or 18. Then 410 pounds of acrylamide from second monomer storage vessel 38 flows through pump 188 through conduits 190 and 192 to the in-line mixer 48.

1,700 pounds (771.11 kg) of a 45% by wt. potassium hydroxide is pumped from storage vessel 22, through pump 80, conduits 82, 84 86 and 88 to one or both potassium hydroxide storage vessels 32 and 34 where the potassium hydroxide solution is further concentrated to a concentration of 53% by wt. and is pumped from vessel 32 through conduits 132, 134 and 136 and pump 138 to potassium hydroxide storage vessel 34 where the potassium hydroxide can be stored before transfer to mixing station 66 through the insulated supply conduit 142 and finally to vessel 12, 14, 16 or 18.

650 pounds (294.84 kg) of potassium carbonate is supplied from vessel 28 to monomer solution mixing vessels 12, 14, 16 and/ or 18 by vibratory feeder 144, through conduit 146, weigh hopper 148, vibratory feeder 150, and turn hed 152 through conduit 154, 156, 158 or 160.

2.9 pounds (1.32 kg) of N, N-methylenebisacrylamide cross-linking agent is weighed on scale 186 and fed manually as indicated diagrammatically at 188 to mixing station 66 for supply to a monomer solution mixing vessel 12, 14, 16 or 18, where the mixed monomer solution is mixed at 110° F. (43.3°C)

The neutralized monomer from the monomer solution mixing vessel 12, 14, 16 or 18 is delivered to the impermeable first belt 52 through in-line mixer 48 where it is combined with a thermal initiator and a redox initiator. The thermal initiator raises the temperature of the mixed monomer solution to 150° F. (65.5°C) The thermal initiator is stored in thermal initiator storage vessels 40 and 42 consisting of a 12% solution of 1,1′-azobis(1-cyclohexanecarbonitrile). 72.2 pounds of thermal initiator would be used for the above batch. The initiator is delivered to the in-line blender 48 through conduit 190, 192 and 194 and pump 199 and, simultaneously 24.1 pounds of 33% t-butylperoxyisopropylcarbonate redox initiator is supplied to the blender 48 through redox initiator storage vessel 44, conduit 200, pump 202 and conduits 204 and 206 for the initiation of the mixed monomer solution.

3.0 pounds (1.36 kg) of a 33% by wt. solution of a 50/50 mixture of sodium thio sulfate and ammonia persulfate redox initiators stored in vessel 44 is fed onto the surface of the polymerizing mixed monomer solution, such as a spray head 210.

### EXAMPLE 8

2,530 pounds (1147.59 kg) of acrylic acid solution from acrylic acid storage vessel 20 at 115°F (46.1°C) and a concentration of 99% by weight flows through valve 60 and pump 62 through conduit 64 to a combining station 66 to vessel 12, 14, 16 or 18. Then 410 pounds (185.97 kg) of styrene from second monomer storage vessel 36 flows through pump 184 through conduits 176 and 178 to monomer weigh tank 180 and is charged to combining station 66 and then to vessel 12, 14 16 or 18, together with the acrylic acid.

1,700 pounds (771.11 kg) of a 45% by wt. potassium hydroxide is pumped from storage vessel 22, through pump 80, conduits 82, 84 86 and 88 to one or both potassium hydroxide storage vessels 32 and 34 where the potassium hydroxide solution is further concentrated to a concentration of 53% by wt. and is pumped from vessel 32 through conduits 132, 134 and 136 and pump 138 to potassium hydroxide storage vessel 34 where the potassium hydroxide can be stored before transfer to mixing station 66 through the insulated supply conduit 142 and finally to vessel 12, 14, 16 or 18.

650 pounds (294.84 kg) of potassium carbonate is supplied from vessel 28 to monomer solution mixing vessels 12, 14, 16 and/or 18 by vibratory feeder 144, through conduit 146, weigh hopper 148, vibratory feeder 150 and turn hed 152 through conduit 154, 156, 158 or 160.

2.9 pounds (1.32 kg) of N, N-methylenebisacrylamide cross-linking agent is weighed on scale 186 and fed manually as indicated diagrammatically at 188 to mixing station 66 for supply to the monomer solution mixing vessel 12, 14, 16 or 18, where the mixed monomer solution is mixed at 110° F. (43.3°C)

The neutralized monomer from the monomer solution mixing vessel 12, 14, 16 or 18 is delivered to the impermeable first belt 52 through in-line mixer 48 where is it combined with a thermal initiator and a redox initiator. The redox initiator raises the temperature of the mixed monomer solution to 150° F. (75.5°C) The thermal initiator is stored in thermal initiator storage vessels 40 and 42 consisting of a 10% solution of (1-phenylethyl)azodiphenylmethane. 72.2 pounds (32.75 kg) of thermal initiator would be used for the above batch. The initiator is delivered to the in-line blender 48 through conduit 190, 192 and 194 and pump 199 and, simultaneously 24.1 pounds (10.93 kg) of 33% peroxy-3,3,5-trimethylcyclohexane redox initiator is supplied to the blender 48 through redox initiator storage vessel 44, conduit 200, pump 202 and conduits 204 and 206 for the initiation of the mixed monomer solution.

3.0 pounds (1.36 kg) of a 33% by wt. solution of a sodium thio sulfate redox initiator stored in vessel 44 is fed onto the surface of the polymerizing mixed monomer solution, such as a spray head 210.

### EXAMPLE 9

2,530 pounds (1147.59 kg) of acrylic acid solution from acrylic acid storage vessel 20 at 110°F (43.3°C) and a concentration of 99% by weight flows through valve 60 and pump 62 through conduit 64 to a combining station 66 to vessel 12, 14, 16 or 18. Then 410 pounds (185.97 kg) of styrene from second monomer storage vessel 36 flows through pump 184 through conduits 176 and 178 to monomer weigh tank 180 and is charged to combining station 66 and then to vessel 12, 14 16 or 18, together with the acrylic acid.

1,700 pounds (771.11 kg) of a 45% by wt. potassium hydroxide is pumped from storage vessel 22, through pump 80, conduits 82, 84 86 and 88 to one or both potassium hydroxide storage vessels 32 and 34 where the potassium hydroxide solution is further concentrated to a concentration of 53% by wt. and is pumped from vessel 32 through conduits 132, 134 and 136 and pump 138 to potassium hydroxide storage vessel 34 where the potassium hydroxide can be stored before transfer to mixing station 66 through the insulated supply conduit 142 and finally to vessel 12, 14, 16 or 18.

650 pounds (294.84 kg) of potassium carbonate is supplied from vessel 28 to monomer solution mixing vessels 12, 14, 16 and/or 18 by vibratory feeder 144, through conduit 146, weigh hopper 148, vibratory feeder 150 and turn hed 152 through conduit 154, 156, 158 or 160.

2.9 pounds (1.32 kg) of N, N-methylenebisacrylamide cross-linking agent is weighed on scale 186 and fed manually as indicated diagrammatically at 188 to mixing station 66 for supply to the monomer solution mixing vessel 12, 14, 16 or 18, where the mixed monomer solution is mixed at 110° F. (43.3°C)

The neutralized monomer from the monomer solution mixing vessel 12, 14, 16 or 18 is delivered to the impermeable first belt 52 through in-line mixer 48 where it is combined with a thermal initiator and a redox initiator. The thermal initiator raises the temperature of the mixed monomer solution to 150° F (66°C). The thermal initiator is stored in thermal initiator storage vessels 40 and 42 consisting of a 12% solution of 2,2'-azobis-(2-methylbutyronitrile). 72.2 pounds of thermal initiator would be used for the above batch. The initiator is delivered to the in-line blender 48 through conduit 190, 192 and 194 and pump 199 and, simultaneously 24.1 pounds of 33% ammonium metabisulfite redox initiator is supplied to the blender 48 through redox initiator storage vessel 44, conduit 200, pump 202 and conduits 204 and 206 for the initiation of the mixed monomer solution.

3.0 pounds (1.36 kg) of a 33% by wt. solution of ammonia persulfate redox initiator stored in vessel 44 is fed onto the surface of the polymerizing mixed monomer solution, such as a spray head 210.

## Claims

1. A method of preparing a solid water absorbing resin which comprises mixing a monomer solution of (A) acrylic acid neutralized 70-100 mole percent; and (B) a water-miscible or water-soluble polyvinyl monomer in a combined concentration of from 30 to 80 wt. %, with water to form a mixed monomer solution, and adding a thermal initiator and a redox initiator to the mixed monomer solution to form an initiated mixed monomer solution, the mixed monomer solution being at a temperature which is below the decomposition temperature of the thermal initiator when the thermal initiator is added thereto, but which is high enough for the addition of the redox initiator to cause sufficient polymerization of the monomers to raise the temperature of the initiated mixed monomer solution to a level such that the thermal initiator decomposes sufficiently to provide, together with the redox initiator, substantially complete polymerization of the monomers.

2. A method according to claim 1, in which the mixed monomer solution is at a temperature of from 30° to 85°C prior to the addition of the initiators thereto.

3. A method according to claim 1 or 2, in which the mixed monomer solution is at a temperature of from 45° to 55°C prior to the addition of the initiators thereto.

4. A method according to any of claims 1 to 3, in which the mixed monomer solution contains from 0.2 to 0.6 wt. % of monomer (B) based on the combined weight of monomers (A) and (B).

5. A method according to any of claims 1 to 4, in which the monomer (B) is N,N-methylenebisacrylamide, N,N-methylenebismethacrylamide, or trimethylolpropane triacrylate.

6. A method according to any of claims 1 to 5, in which the mixed monomer solution contains 1 to 10 wt. % of an organic solvent based on the weight of monomers (A) and (B).

7. A method according to any of claims 1 to 6, in which polymerization is effected such that less than 1000 PPM of free acrylic monomer remains in the polymerized resin.

8. A method according to any of claims 1 to 7, in which polymerization is effected such that less than 200 PPM of free acrylic monomer remains in the polymerized resin.

9. A method according to any of claims 1 to 8, in which, during polymerization, the exothermic heat of reaction is substantially the only heat energy used to accomplish polymerization, cross-linking and to drive off sufficient water to obtain a solid cross-linked resin having a water content of not more than 15 percent by weight.

10. A method according to any of claims 1 to 9 which is carried out continuously and in which the mixed monomer solution is combined with the thermal initiator and the redox initiator in a mixing chamber (48), the resulting mixture is supplied to a polymerization station (50) for polymerization, the polymerization station being simultaneously continuously supplied with additional mixed monomer solution and additional polymerization initiators, and solid polyacrylate resin is continuously removed from the polymerization station.

11. A method according to claim 10, in which the polymerization station (50) comprises an endless conveyor belt (52).

12. A method according to claim 10 or 11, in which the mixed monomer solution and the polymerization initiators are supplied to the mixing chamber (48) at rates such that the combined concentration of the initiators is continuously at least 0.5 wt. % with respect to the monomers (A) and (B).

13. A method according to any of claims 10 to 12, in which the solid resin obtained is reduced to a powder.

## Patentansprüche

1. Verfahren zum Herstellen eines festen wasserabsorbierenden Harzes, welches umfaßt das Mischen einer Monomerlösung aus (A) Acrylsäure neutralisiert 70 - 100 Mol-%; und (B) einem wassermischbaren oder wasserlöslichen Polyvinylmonomer in einer kombinierten Konzentration von 30 bis 80 Gew.-%, mit Wasser, um eine gemischte Monomerlösung zu bilden, und das Zugeben eines thermischen Initiators und eines Redoxinitiators zu der gemischten Monomerlösung, um eine initiierte gemischte Monomerlösung zu bilden, wobei die gemischte Monomerlösung sich bei einer Temperatur befindet, die unterhalb der Zersetzungstemperatur des thermischen Initiators liegt, wenn der thermische Initiator dazugegeben wird, aber die hoch genug ist, daß die Zugabe des Redoxinitiators eine ausreichende Polymerisation der Monomeren verursacht, so daß die Temperatur der initiierten gemischten Monomerlösung auf einen Wert angehoben wird, so daß der thermische Initiator in ausreichender Weise sich zersetzt, um zusammen mit dem Redoxinitiator die im wesentlichen vollständige Polymerisation der Monomeren zu ergeben.

2. Verfahren nach Anspruch 1, worin die gemischte Monomerlösung bei einer Temperatur von 30 ° bis 85 °C vor der Zugabe der Initiatoren dazu ist.

3. Verfahren nach Anspruch 1 oder 2, worin die gemischte Monomerlösung bei einer Temperatur von 45 ° bis 55 °C vor der Zugabe der Initiatoren dazu ist.

4. Verfahren nach einem der Ansprüche 1 bis 3, worin die gemischte Monomerlösung von 0,2 bis 0,6 Gew.-% Monomer (B) bezogen auf das kombinierte Gewicht der Monomeren (A) und (B) enthält.

5. Verfahren nach einem der Ansprüche 1 bis 4, worin das Monomer (B) N,N-Methylenbisacrylamid, N,N-Methylenbismethacrylamid oder Trimethylolpropantriacrylat ist.

6. Verfahren nach einem der Ansprüche 1 bis 5, worin die gemischte Monomerlösung 1 bis 10 Gew.-% eines organischen Lösungsmittels bezogen auf das Gewicht der Monomeren (A) und (B) enthält.

7. Verfahren nach einem der Ansprüche 1 bis 6, worin die Polymerisation so bewirkt wird, daß weniger als 1000 ppm an freiem Acrylmonomer in dem polymerisierten Harz verbleiben.

8. Verfahren nach einem der Ansprüche 1 bis 7, worin die Polymerisation so bewirkt wird, daß weniger als 200 ppm an freiem Acrylmonomer in dem polymerisierten Harz verbleiben.

9. Verfahren nach einem der Ansprüche 1 bis 8, worin während der Polymerisation die exotherme Reaktionswärme im wesentlichen die einzige Wärmeenergie ist, die verwendet wird, um die Polymerisation, Vernetzung und das Austreiben von ausreichend viel Wasser zum Erhalten eines festen vernetzten Harzes mit einem Wassergehalt von nicht mehr als 15 Gew.-% zu erreichen.

10. Verfahren nach einem der Ansprüche 1 bis 9, welches kontinuierlich durchgeführt wird und in dem die gemischte Monomerlösung mit dem thermischen Initiator und dem Redoxinitiator in einer Mischkammer (48) vereinigt wird, die resultierende Mischung einer Polymerisationsstation (50) zur Polymerisation zugeführt wird, wobei die Polymerisationsstation gleichzeitig kontinuierlich mit zusätzlicher gemischter Monomerlösung und zusätzlichen Polymerisationsinitiatoren versorgt wird, und festes Polyacrylatharz kontinuierlich von der Polymerisationsstation entnommen wird.

11. Verfahren nach Anspruch 10, worin die Polymerisationsstation (50) ein endloses Förderband (52) umfaßt.

12. Verfahren nach Anspruch 10 oder 11, worin die gemischte Monomerlösung und die Polymerisationsinitiatoren der Mischkammer (48) mit solchen Geschwindigkeiten zugeführt werden, daß die kombinierte Konzentration der Initiatoren kontinuierlich mindestens 0,5 Gew.-% bezüglich der Monomeren (A) und (B) beträgt.

13. Verfahren nach einem der Ansprüche 10 bis 12, in dem das erhaltene feste Harz in ein Pulver überführt wird.

## Revendications

1. Procédé de préparation d'une résine solide absorbant l'eau, comprenant le mélange d'une solution monomère de (A) acide acrylique neutralisé à 70 à 100 % en moles et (B) un monomère polyvinylique miscible à l'eau ou soluble dans l'eau à une concentration combinée comprise entre 30 et 80 % en poids, avec de l'eau pour former une solution de monomères mélangés et l'addition d'un amorceur thermique et d'un amorceur d'oxydoréduction à la solution de monomères mélangés pour former une solution amorcée de monomères mélangés, la solution de monomères mélangés étant à une température inférieure à la température de décomposition de l'amorceur thermique lorsque l'amorceur thermique lui est ajouté, mais suffisamment élevée pour que l'addition de l'amorceur d'oxydoréduction induise une polymérisation des monomères suffisante pour élever la température de la solution amorcée de monomères mélangés à un niveau tel que l'amorceur thermique se décompose suffisamment pour assurer, avec l'amorceur d'oxydoréduction, une polymérisation sensiblement complète des monomères.

2. Procédé selon la revendication 1, dans lequel la solution de monomères mélangés se trouve à une température comprise entre 30° et 85°C avant l'addition des amorceurs.

3. Procédé selon la revendication 1 ou 2, dans lequel la solution de monomères mélangés se trouve à une température comprise entre 45° et 55°C avant l'addition des amorceurs.

4. Procédé selon l'une quelconque des revendications 1 à 3, dans lequel la solution de monomères mélangés contient entre 0,2 et 0,6 % en poids de monomère (B) par rapport au poids combiné des monomères (A) et (B).

5. Procédé selon l'une quelconque des revendications 1 à 4, dans lequel le monomère (B) est le N,N-méthylène-bis-acrylamide, le N,N-méthylène-bis-méthacrylamide, ou le triacrylate de triméthylolpropane.

6. Procédé selon l'une quelconque des revendications 1 à 5, dans lequel la solution de monomères mélangés contient 1 à 10 % en poids d'un solvant organique par rapport au poids des monomères (A) et (B).

7. Procédé selon l'une quelconque des revendications 1 à 6, dans lequel la polymérisation est effectuée de façon que moins de 1 000 ppm du monomère acrylique libre demeurent dans la résine polymérisée.

8. Procédé selon l'une quelconque des revendications 1 à 7, dans lequel la polymérisation est effectuée de façon que moins de 200 ppm du monomère acrylique libre demeurent dans la résine polymérisée.

9. Procédé selon l'une quelconque des revendications 1 à 8, dans lequel, durant la polymérisation, la chaleur exothermique de la réaction est sensiblement la seule énergie calorifique utilisée pour procéder à la polymérisation et à la réticulation et pour éliminer suffisamment d'eau pour obtenir une résine solide réticulée ayant une teneur en eau inférieure ou égale à 15 % en poids.

10. Procédé selon l'une quelconque des revendications 1 à 9, lequel est opéré en continu et dans lequel la solution de monomères mélangés est combinée à l'amorceur thermique et à l'amorceur d'oxydoréduction dans une chambre de mélange (48), le mélange obtenu est amené à un poste de polymérisation (50) en vue de la polymérisation, le poste de polymérisation étant simultanément alimenté en continu en solution additionnelle de monomères mélangés et en initiateurs additionnels de polymérisation, et la résine de polyacrylate solide est retirée en continu du poste de polymérisation.

11. Procédé selon la revendication 10, dans lequel le poste de polymérisation (50) comprend une bande convoyeuse sans fin (52).

12. Procédé selon la revendication 10 ou 11, dans lequel la solution de monomères mélangés et les amorceurs de polymérisation sont amenés à la chambre de mélange (48) à des vitesses telles que la concentration combinée des initiateurs est constamment supérieure ou égale à 0,5 % en poids par rapport aux monomères (A) et (B).

13. Procédé selon l'une quelconque des revendications 10 à 12, dans lequel la résine solide obtenue est réduite en poudre.
